# EUROPÄISCHE PATENTANMELDUNG

(11) **EP 0 829 224 A2**
(43) Veröffentlichungstag der Anmeldung: **18.03.1998**
(21) Anmeldenummer: 97114114.8
(22) Anmeldetag: 14.08.1997
(51) Int. Cl.: A61B 5/00

(54) **Differentieller Messkopf für die photoakustische Spektroskopie**

(30) Priorität: 14.08.1996 DE 19632864
(71) Anmelder: Columbus Schleif-und Zerspantechnik Hard-und Software GmbH, 87634 Obergünzburg (DE)
(72) Erfinder: Zürl, K. Dr.Ing., 87435 Kempten (DE); Weiss, Armin, 82467 Garmisch-Partenkirchen (DE); Zobel, F., 87496 Untrasried (DE); Boos, Karl-Siegfried Prof., 82131 Gauting (DE)
(74) Vertreter: VOSSIUS & PARTNER

(57) **Zusammenfassung**

Es wird ein differentieller Meßkopf für photoakustische Messungen zur Verfügung gestellt, der zwei oder mehrere Meßzellen enthält und wobei intensitätsmoduliertes Licht gleicher Wellenlängen in jeweils zwei benachbarten Meßzellen eingestrahlt wird. Die Vorteile der Erfindung liegen in einer höheren Empfindlichkeit für die zu messende Größe bei gleichzeitiger Immunität gegen Störungen von außen, in der Festellung und Kompensation von Inhomogenitäten in der Probenoberfläche z.B. in der Haut des Menschen und in einem vergrößerten Dynamikbereich.

## Beschreibung

Die Erfindung betrifft einen differentiellen Meßkopf für die photoakustische Spektroskopie, der bevorzugt in der medizinischen Meßtechnik am menschlichen Körper eingesetzt wird.

Die photoakustische Spektroskopie (PAS) ist ein spektroskopisches Verfahren, zur Bestimmung strahlungsabsorbierender Inhaltsstoffe in gasförmigen, flüssigen oder festen Proben.

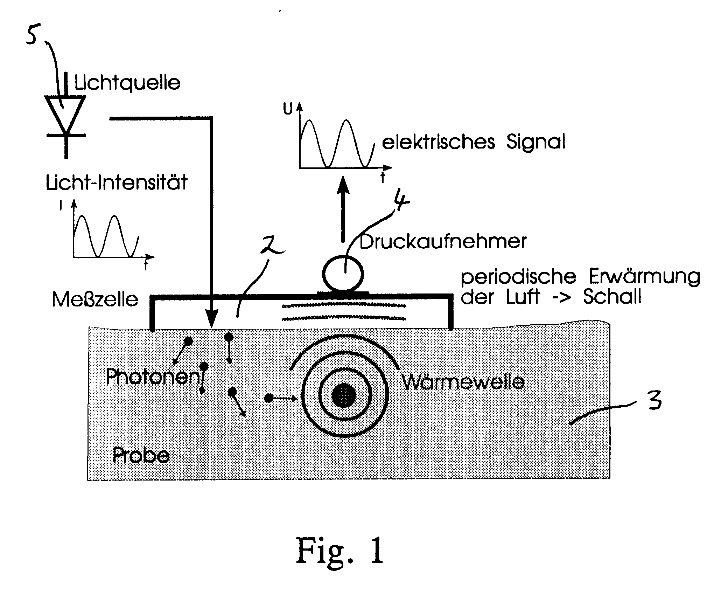

Fig. 1 stellt das Meßprinzip der PAS schematisch dar.

Bei der Einstrahlung von Licht aus einer Lichtquelle 5 auf eine Probe 3 wird die Probe erwärmt, falls die Lichtstrahlung durch Probenbestandteile absorbiert wird. Die Erwärmung breitet sich bis zur Oberfläche der Probe 3 aus und wird in die an die Probenoberfläche angrenzende Luftschicht übertragen. Ist die Intensität der einfallenden Strahlung periodisch moduliert, so ergibt sich eine periodische Erwärmung der Luftschicht über der Probe 3 und damit eine periodische Druckschwankung, d.h. eine Schallwelle in einer auf der Probenoberfläche aufliegenden Meßzelle 2. Die Amplitude dieser Schallwelle ist ein Maß für die in der Probe 3 absorbierte Lichtintensität, also für die Konzentration der strahlungsabsorbierenden Inhaltsstoffe.

Bei der PAS treten folgende Probleme auf.

Je nach Ort der Messung treten oft starke Störungen durch Geräusche, die nicht auf photoakustischem Weg erzeugt wurden, z.B. Umgebungsgeräusche, Körperschall, bei Messungen am menschlichen Körper, Geräusche durch Atmung, Bewegungen oder den Blutstrom auf. Dabei ist es auch bei Einsatz von angepaßten Signalverarbeitungstechniken wie z.B. Lock-In-Verstärkertechnik, oft schwierig, Nutz- und Störsignale zu unterscheiden. Die Zuverlässigkeit der Messungen wird folglich durch die Stärke der Störgeräusche begrenzt. Bei zu starken Störgeräuschen besteht auch die Gefahr, daß der Dynamikumfang des verwendeten Schallaufnehmers überschritten wird und die Messungen aus diesem Grund unbrauchbare Ergebnisse liefern. Weiterhin ist ein dichtes Anliegen des Meßkopfes an der Probenoberfläche wichtig. Kleinste Undichtigkeiten können das Meßergebnis verfälschen. Es wurde jedoch bislang keine brauchbare Methode beschrieben, die einen korrekten Sitz des Meßkopfes gewährleistet oder den Sitz des Meßkopfes überprüft. Bei der PAF *in vivo* am menschlichen Körper, z.B. zur Messung von Blutinhaltsstoffen ist die Stärke des photoakustischen Signals durch die erlaubte Bestrahlungsstärke auf die Haut (DIN Norn EN 60825-1) begrenzt. Bei der PAS *in vivo* am menschlichen Körper soll möglichst das Meßgerät erkennen, ob der Meßkopf auf einer Körperpartie aufsitzt, deren Hautstruktur sehr inhomogen ist (Leberflecken, Altersflecken). Damit wäre eine automatische Beurteilung der Relevanz der Meßwerte während des Meßvorgangs möglich. Jedoch wurde eine solche Methode bisher noch nicht beschrieben.

Aus der US-A-5 452 716 ist ein photoakustischer Meßkopf mit zwei oder mehreren Meßzellen bekannt. Die Meßzellen sind paarweise durch Druckaufnehmer verbunden. In benachbarten Meßzellen wird Licht verschiedener Wellenlängen im Wellenlängenbereich von 1 bis 40 µm eingestrahlt. Mit diesem photoakustischen Meßkopf ist eine Messung von Signaldifferenzen, jedoch keine Messung von absoluten Signalgrößen möglich.

Aus der FR-A-2 728 452 und aus der JP-A-1-191040 ist eine ähnliche Vorrichtung bekannt, bei der in zwei Kammern Licht derselben Wellenlänge eingestrahlt wird. Jedoch sind diese Kammern nicht mit einem Differenzdruckaufnehmer verbunden, sondern weisen zwei separate Aufnehmer auf.

Bei dem System gemäß P. Poulet, J.E.J. Chambron, Med. & Biol. Eng. & Comp. 23, 585 (1985) ist zwar ein Differenzdruckaufnehmer zwischen benachbarten Kammern vorhanden, jedoch wird nur in eine Kammer Licht eingestrahlt.

Der Erfindung liegt die Aufgabe zugrunde, einen differentiellen Meßkopf für die photoakustische Spektroskopie zur Verfügung zu stellen, wobei die genannten Nachteile im Stand der Technik überwunden werden.

Die Aufgabe wird mit den Merkmalen der Patentansprüche gelöst.

Vorteilhafterweise ermöglicht die Erfindung eine höhere Empfindlichkeit für die zu messende Größe bei gleichzeitiger Immunität gegen Störungen, die Feststellung und Kompensation von Inhomogenitäten auf der Probenoberfläche (z.B. der Haut) und einen großen Dynamikbereich der Messung.

Im folgenden wird die Erfindung anhand der Zeichnungen näher erläutert. Es zeigen:
- Fig. 1: eine schematische Darstellung des Meßprinzips der PAS,
- Fig. 2: eine erste erfindungsgemäße Ausführungsform,
- Fig. 3: eine zweite erfindungsgemäße Ausführungsform,
- Fig. 4: eine dritte erfindungsgemäße Ausführungsform, und
- Fig. 5: eine vierte erfindungsgemäße Ausführungsform.

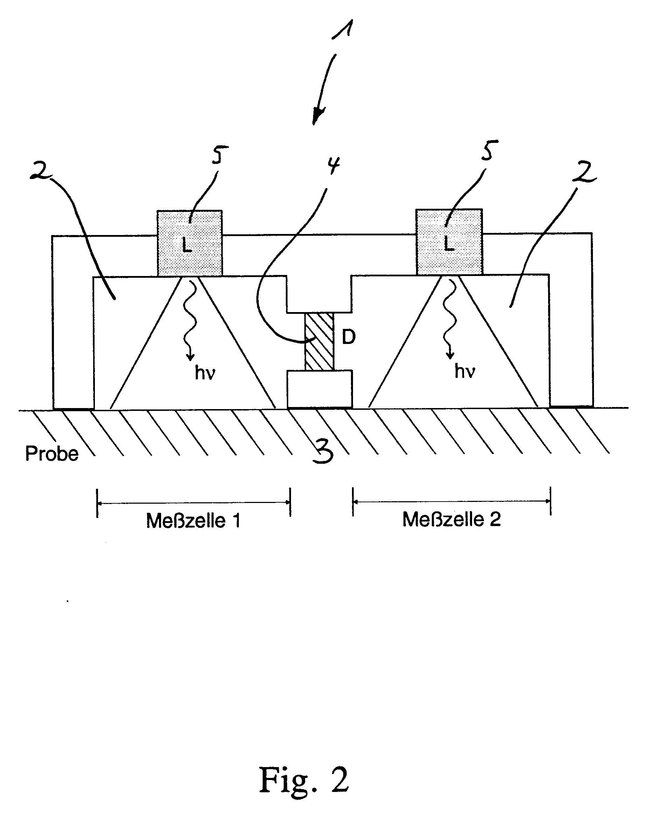

Eine erste erfindungsgemäße Ausführungsform ist in Fig. 2 dargestellt. Der Meßkopf 1 besteht aus zwei Meßzellen 2, die durch einen Druckaufnehmer 4 miteinander verbunden sind. Die Lichtquellen 5 strahlen Licht gleicher Wellenlänge mit gegenphasiger Modulation in beide Meßzellen ein. Es wird eine akustische Kompensation der Störgeräusche, d.h. eine Kompensation vor Ansprechen des Druckaufnehmers 4 erreicht, unter der Voraussetzung, daß die Störsignale gleichermaßen auf die beiden Meßzellen 2 wirken. Somit werden die Anforderungen an den Dynamikbereich des Druckaufnehmers reduziert. Durch die Einstrahlung von Licht gleicher Wellenlänge bei gegenphasiger Modulation kann gegenüber einer Doppelkammermethode mit unbestrahlter Referenzzelle eine Verdopplung des Nutzsignals erreicht werden. Bei Einstrahlung von Licht gleicher Wellenlänge und gleichphasiger Modulation in beide Meßzellen 2 können Oberflächeninhomogenitäten der Probe 3 (z.B. Materialverschiedenheiten bzw. bei Messung am menschlichen Körper Hautinhomogenitäten erkannt werden. Durch Kombination von gleichphasiger und gegenphasiger Modulation kann eine Messung, bei der zuerst der Probenbereich auf mögliche Oberflächeninhomogenität untersucht wird und danach eine Messung von Probeninhaltsstoffen erfolgt, durchgeführt werden. Dabei wird in zwei durch einen Druckaufnehmer verbundene Meßzellen Licht gleicher Wellenlänge zuerst mit gleichphasiger und anschließend mit gegenphasiger Modulation eingestrahlt.

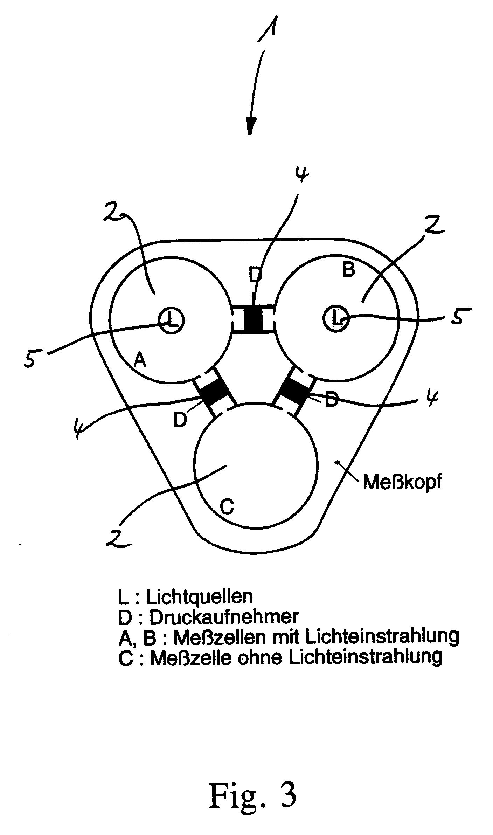

In Fig. 3 ist ein Meßkopf mit drei Meßzylindern dargestellt. Die drei Meßzylinder 2 sind paarweise durch Druckaufnehmer 4 verbunden. Mit diesem Meßkopf kann die Untersuchung auf mögliche Oberflächeninhomogenitäten und die Messung von Probeninhaltsstoffen gleichzeitig stattfinden. In zwei Meßzellen 2A und 2B wird dabei gegenphasig eingestrahlt, und die dritte Meßzelle 2C sitzt auf der unbestrahlten Probenoberfläche. Der Schalldruck zwischen den beiden Meßzellen 2A und 2B, in die die Einstrahlung erfolgt, ergibt dabei den gesuchten Meßwert. Die Differenzen zwischen den bestrahlten Meßzellen und der unbestrahlten Zelle 2C werden verglichen, wodurch Inhomogenitäten der Probenoberfläche unter den beiden bestrahlten Meßzellen erkannt werden können. In dieser Ausführungsform wird der Meßkopf gegenüber der ersten Ausführungsform um eine Meßzelle erweitert, es wird aber Meßzeit gespart.

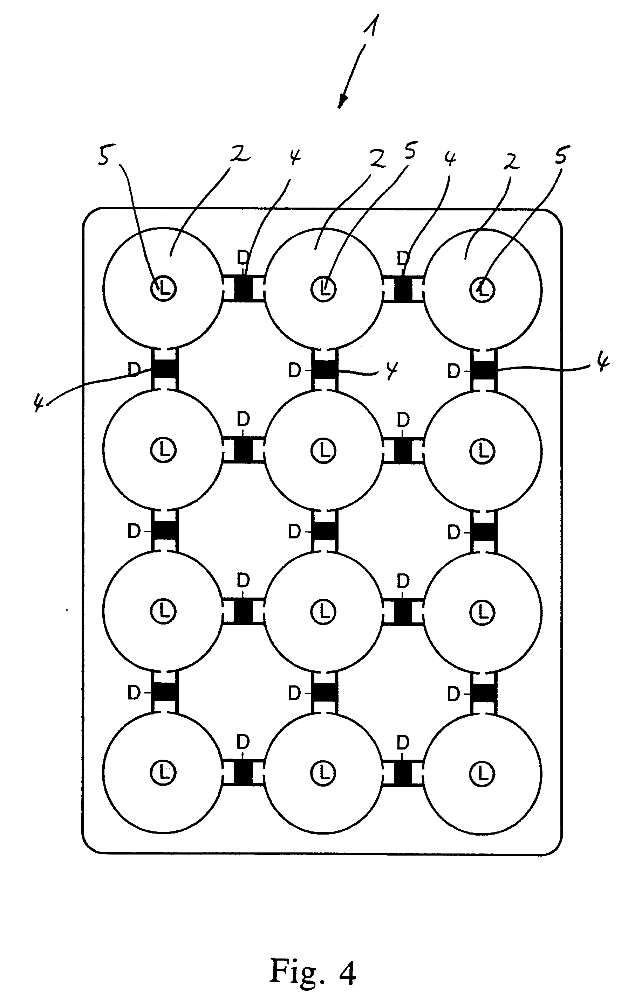

Ein Meßkopf mit mehr als drei Meßzellen, die in Form eines Rasters, z.B. eines Gitters angeordnet und mit den jeweils benachbarten Meßzellen 2 durch Druckaufnehmer 4 verbunden sind, ist in Fig. 4 dargestellt. Mit dieser dritten erfindungsgemäßen Ausführungsform ist eine bildgebende PAS zur Charakterisierung von Probenoberflächen möglich. Jede Meßzelle 2 weist dabei eine eigene Lichtquelle 5 auf. Es muß jedoch nicht für jede Meßzelle 2 eine Lichtquelle zur Verfügung stehen. Es kann auch die Strahlung einer oder weniger Lichtquellen durch geeignete Lichtleitsysteme z.B. Lichtleitfasern oder Dammann-Gitter auf die Meßzellen 2 verteilt werden. Werden mit Hilfe eines Meßkopfs gemäß der dritten erfindungsgemäßen Ausführungsform Messungen bei mehreren Modulationsfrequenzen durchgeführt, kann zusätzlich zu der lateralen eine Tiefen-Auflösung der PAS erreicht werden. Damit könnte z.B. in der Medizin die räumliche Ausdehnung von Geschwulsten oder in der Materialprüfung die räumliche Ausdehnung von Einschlüssen oder Lunkern nachgewiesen werden.

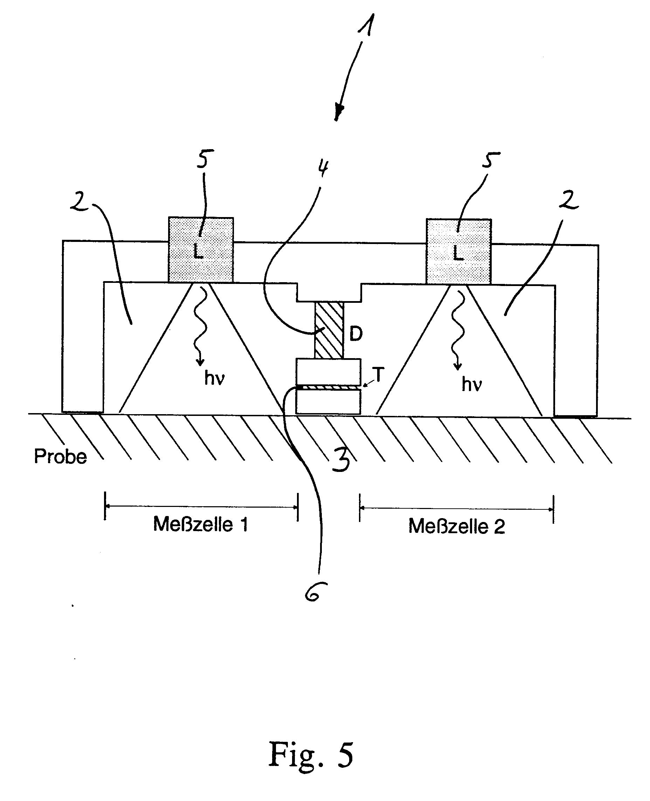

Eine vierte erfindungsgemäße Ausführungsform zeigt Fig. 5. Zur Erreichung eines optimalen Sitz des Meßkopfs 1 auf der Oberfläche der Probe 3 wird ein Unterdruck in den Meßzellen 2 angelegt. Dabei muß der Unterdruck in jeweils zwei durch einen Druckaufnehmer 4 verbundenen Meßzellen 2 gleich sein, um die korrekte Funktion des Druckaufnehmers zu gewährleisten. Dieses Verfahren kann bei der PAS mit nur einer Meßzelle nicht angewandt werden, da der Druckaufnehmer in diesem Falle immer gegen ein Gasvolumen mit konstantem Druck z.B. gegen den umgebenden atmosphärischen Druck arbeiten muß. Ein gleicher Unterdruck in mehreren Meßzellen 2 wird durch ein Element 6 erreicht, das als akustischer Tiefpaß wirkt. Das Element 6 besteht aus Luftkanälen, die einen langsamen Luftausgleich zulassen, jedoch so dimensioniert sind, daß sie für Druckschwankungen mit der Frequenz des photoakustischen Signals als Sperre wirken. Durch zusätzliche Druckaufnehmer, die gegen den umgebenden atmosphärischen Druck arbeiten, kann die Aufrechterhaltung des Unterdrucks während der Messung und damit der korrekte Sitz des Meßkopfs 1 kontrolliert werden.

Die Erfindung läßt sich für die Analytik von Gasen Flüssigkeiten und Festkörpern einsetzen.

Dabei können diskontinuierliche, kontinuierliche, qualitative und quantitative Messungen vorgenommen werden.

Die Erfindung ermöglicht die nicht-invasive d.h. zerstörungs- und kontimationsfreie Bestimmung von Stoffen in biologischen Flüssigkeiten wie Vollblut, Plasma, Serum, Milch oder Urin, in Prozeßflüssigkeiten, in Oberflächen-, Trink- und Abwasser, in menschlichen, tierischen, pflanzlichen und bakteriellen Zellverbänden bzw. deren Suspensionen oder Homogenate, z.B. Fermenterbrühen und Zellkulturen, in menschlichen, tierischen und pflanzlichen Geweben oder Organen, in Nahrungsmitteln, Arzneimitteln und bei histochemischen, zellbiologischen, immunologischen, molekularbiologischen und klinisch-chemischen Analysenverfahren.

Ein bevorzugtes Anwendungsgebiet ist die nicht-invasive Bestimmung von Blutzucker über die menschliche Haut.

## Patentansprüche

1. Meßkopf (1) für photoakustische Messungen, insbesondere zum gleichzeitigen Aufsetzen auf die Oberfläche einer Probe (3), wobei der Meßkopf (1) zwei oder mehrere Meßzellen (2) enthält und benachbarte Meßzellen (2) untereinander durch einen oder mehrere Druckaufnehmer (4) verbunden sind, dadurch gekennzeichnet, daß in die Meßzellen (2) intensitätsmoduliertes Licht gleicher Wellenlängen eingestrahlt wird.

2. Differentieller Meßkopf (1) für photoakustische Messungen, der wenigstens zwei Meßzellen (2) enthält, die zum direkten Aufsetzen auf die Oberfläche einer Probe (3) einseitig offen sind, und wobei benachbarte Meßzellen (2) untereinander derart durch einen oder mehrere Druckaufnehmer (4) verbunden sind, daß die Druckaufnehmer (4) die Druckdifferenz der benachbarten Meßzellen erfassen, dadurch gekennzeichnet, daß in wenigstens zwei derart durch einen Druckaufnehmer (4) verbundene Meßzellen (2) intensitätsmoduliertes Licht gleicher Wellenlänge eingestrahlt wird.

3. Meßkopf nach Anspruch 1 oder 2, dadurch gekennzeichnet, daß Licht im Wellenlängenbereich von 400 bis 2500 nm eingestrahlt wird.

4. Meßkopf nach Anspruch 1, 2 oder 3, dadurch gekennzeichnet, daß in jeweils zwei benachbarten Meßzellen (2) gegenphasig intensitätsmoduliertes Licht eingestrahlt wird.

5. Meßkopf nach Anspruch 1, 2 oder 3, dadurch gekennzeichnet, daß in jeweils zwei benachbarte Meßzellen gleichphasig intensitätsmoduliertes Licht eingestrahlt wird.

6. Meßkopf nach Anspruch 1 oder 2, dadurch gekennzeichnet, daß er drei Meßzellen (2) aufweist, die durch zwei oder drei Druckaufnehmer (4) untereinander verbunden sind, wobei in zwei (2A, 2B) der drei Meßzellen gegenphasig intensitätsmoduliertes Licht gleicher Wellenlänge eingestrahlt wird und in die dritte Meßzelle (2C) keine Lichteinstrahlung erfolgt.

7. Meßkopf nach Anspruch 1 oder 2, dadurch gekennzeichnet, daß er mehrere Meßzellen (2) aufweist, die in Form eines Rasters oder Gitters angeordnet und mit jeweils mindestens zwei benachbarten Meßzellen (2) durch Druckaufnehmer (4) verbunden sind.

8. Meßkopf nach einem der Ansprüche 1 bis 7, dadurch gekennzeichnet, daß jede Meßzelle (2) eine Lichtquelle (5) enthält.

9. Meßkopf nach einem der Ansprüche 1 bis 8, dadurch gekennzeichnet, daß der Meßkopf (1) mindestens ein Lichtleitsystem aufweist, das die Strahlung einer oder mehrerer Lichtquellen auf die einzelnen Meßzellen (2) verteilt.

10. Meßkopf nach Anspruch 9, dadurch gekennzeichnet, daß das Lichtleitsystem die Strahlung zweier gegenphasig intensitätsmodulierter Lichtquellen auf die einzelnen Meßzellen verteilt, so daß die in jeweils zwei benachbarte Meßzellen (2) eingestrahlte Strahlung gegenphasig intensitätsmoduliert ist.

11. Meßkopf nach Anspruch 9, dadurch gekennzeichnet, daß das Lichtleitsystem die Strahlung einer intensitätsmodulierten Lichtquelle auf die einzelnen Meßzellen (2) verteilt, so daß die in allen Meßzellen (2) eingestrahlte Strahlung gleichphasig intensitätsmoduliert ist.

12. Meßkopf nach einem der Ansprüche 9 bis 11, dadurch gekennzeichnet, daß das Lichtleitsystem unter Verwendung von Lichtleitfasern aufgebaut ist.

13. Meßkopf nach einem der Ansprüche 8 bis 11, dadurch gekennzeichnet, daß das Lichtleitsystem unter Verwendung von Dammann-Gittern aufgebaut ist.

14. Meßkopf nach einem der Ansprüche 1 bis 13, dadurch gekennzeichnet, daß in den einzelnen Meßzellen (2) ein Unterdruck erzeugt wird, um einen optimalen Sitz des Meßkopfs (1) auf der Probe (3) zu gewährleisten.

15. Meßkopf nach Anspruch 14, dadurch gekennzeichnet, daß zwischen den durch Druckaufnehmern (4) verbundenen Meßzellen (2) Elemente (6) angebracht sind, die als akustische Tiefpässe wirken, die einen langsamen Druckausgleich zulassen und einen schnellen Druckausgleich nicht zulassen.

16. Meßkopf nach Anspruch 15, dadurch gekennzeichnet, daß das Element (6) durch Luftkanäle zwischen den Meßzellen (2) gebildet wird, die einen langsamen Druckausgleich zulassen, jedoch so dimensioniert sind, daß sie für Druckschwankungen mit der Frequenz des photoakustischen Signals als Sperre wirken.

17. Meßkopf nach einem der Ansprüche 14 bis 16, dadurch gekennzeichnet, daß die Meßzellen (2) zusätzliche Druckaufnehmer enthalten, die den Druck in den betreffenden Meßzellen relativ zum umgebenden atmosphärischen Druck messen.

18. Verwendung eines Meßkopfs nach einem der Ansprüche 1 bis 17 zur nicht-invasiven Bestimmung von Stoffen in biologischen Flüssigkeiten wie Vollblut, Plasma, Serum, Milch oder Urin; in Prozeßflüssigkeiten; in Oberflächen-, Trink- und Abwasser-; in menschlichen, tierischen, pflanzlichen und bakteriellen Zellverbänden bzw. deren Suspensionen oder Homogenate wie Fermenterbrühen, oder Zellkulturen; in menschlichen, tierischen und pflanzlichen Geweben und Organen; Nahrungsmitteln, Arzneimitteln; und im Rahmen von histochemischen, zellbiologischen, immunologischen, molekularbiologischen und klinisch-chemischen Analysenverfahren.

19. Verwendung nach Anspruch 18 für Messungen an der menschlichen Körperoberfläche zur nicht-invasiven Bestimmung der Blutzuckerkonzentration.

20. Verwendung des Meßkörpers nach einem der Ansprüche 1 bis 17 zur Analytik von Festkörpern oder Gasen.
